(19) Europäisches Patentamt
European Patent Office
Office européen des brevets

(11) EP 1 414 405 B1

(12) FASCICULE DE BREVET EUROPEEN

(45) Date de publication et mention de la délivrance du brevet:
**04.07.2007 Bulletin 2007/27**

(21) Numéro de dépôt: **02791424.1**

(22) Date de dépôt: **18.07.2002**

(51) Int Cl.:
***A61K 8/06*** *(2006.01)* ***A61K 8/63*** *(2006.01)*
***A61K 8/898*** *(2006.01)* ***A61K 31/56*** *(2006.01)*
***A61Q 19/00*** *(2006.01)* ***A61Q 19/02*** *(2006.01)*
***A61Q 19/08*** *(2006.01)* ***A61Q 5/00*** *(2006.01)*
***A61Q 5/10*** *(2006.01)*

(86) Numéro de dépôt international:
**PCT/FR2002/002569**

(87) Numéro de publication internationale:
**WO 2003/011243 (13.02.2003 Gazette 2003/07)**

(54) **EMULSION INVERSE CONTENANT DE LA DHEA**

INVERSE EMULSION MIT DHEA

INVERT EMULSION CONTAINING DHEA

(84) Etats contractants désignés:
**AT BE BG CH CY CZ DE DK EE ES FI FR GB GR IE IT LI LU MC NL PT SE SK TR**
Etats d'extension désignés:
**LT LV RO SI**

(30) Priorité: **02.08.2001 FR 0110398**

(43) Date de publication de la demande:
**06.05.2004 Bulletin 2004/19**

(73) Titulaire: **GALDERMA RESEARCH & DEVELOPMENT**
**06410 Biot (FR)**

(72) Inventeurs:
- **ASTRUC, Fanny**
  **F-06200 Nice (FR)**
- **ORSONI, Sandrine**
  **F-06210 Mandelieu (FR)**
- **FREDON, Laurent**
  **F-06330 Roquefort les Pins (FR)**
- **SIMONNET, Jean-Thierry**
  **F-75011 Paris (FR)**
- **RICHART, Pascal**
  **F-75013 Paris (FR)**

(74) Mandataire: **Andral, Christophe André Louis**
**L'OREAL - D.I.P.I.**
**25-29 Quai Aulagnier**
**92600 Asnières (FR)**

(56) Documents cités:
**EP-A- 0 587 288** **WO-A-01/26618**
**FR-A- 2 777 194** **FR-A- 2 803 513**

- **DATABASE WPI Week 198540 Derwent Publications Ltd., London, GB; AN 1985-246143 XP002200433 & JP 60 161912 A (KANEBO)**

EP 1 414 405 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

L'invention concerne une nouvelle composition de type émulsion inverse contenant de la DHEA et/ou ses précurseurs ou dérivés chimiques et/ou biologiques, et ses utilisations en cosmétique et en dermatologie

La peau humaine est constituée de deux compartiments, à savoir un compartiment profond, le derme, et un compartiment superficiel, l'épiderme.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale. On y trouve aussi des leucocytes, des mastocytes ou encore des macrophages tissulaires. Il contient également des vaisseaux sanguins et des fibres nerveuses.

L'épiderme est en contact avec l'environnement extérieur. Son rôle consiste à protéger l'organisme de la déshydratation et des agressions extérieures, qu'elles soient chimiques, mécaniques, physiques ou infectieuses.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Les cellules constituant l'épiderme sont délimitées par un domaine lipidique. Les lipides épidermiques sont synthétisés principalement dans l'épiderme vivant. Ils sont essentiellement constitués de phospholipides, de sphingolipides, de cholestérol, d'acides gras libres, de triglycérides, d'esters du cholestérol et d'alcanes. Au cours de la différenciation cellulaire, les phospholipides dont le rôle consiste à élaborer la structure fluide des membranes cellulaires des couches vivantes de l'épiderme, sont peu à peu remplacés par un mélange composé en majeure partie d'acides gras, de cholestérol et de sphingolipides, constituants essentiels de la couche cornée de l'épiderme (stratum corneum).

Les lipides du ciment inter-cornéocytaire de la peau, et notamment les céramides, sont organisés en bi-couches lamellaires ou feuillets et participent à la cohésion du stratum corneum en vue de maintenir l'intégrité de la barrière et son rôle protecteur, antipénétration et anti-irritation notamment.

On comprend qu'une activation du métabolisme au niveau des cellules vivantes de l'épiderme, ou un accroissement de la prolifération cellulaire au niveau des couches vivantes, se traduiront par une augmentation du contenu de l'épiderme en phospholipides (sphingomyéline I phosphatidylinositol ou phospholipides membranaires, respectivement) et résulteront en une augmentation de la taille ou du nombre des cellules vivantes, c'est-à-dire en un épaississement de l'épiderme.

Cette activation physiologique permettra ainsi prévenir ou de lutter contre les signes du vieillissement chronologique ou actinique ainsi que contre certaines pathologies cutanées.

En effet, on sait qu'au cours du vieillissement chronobiologique, en particulier à la ménopause, on observe une atrophie de l'épiderme résultant d'un ralentissement général du métabolisme cellulaire et qui est pour partie responsable de l'apparition de rides et ridules. L'atrophie de l'épiderme a également été identifiée comme l'un des signes histologiques du photovieillissement (Gilchrest B.A., Skin and Aging Processes, 1989, CRC Press).

Ce processus est également présent au niveau d'autres muqueuses, notamment lors de l'atrophie vulvaire ou vaginale.

On comprend donc l'intérêt de disposer d'un moyen facilitant la multiplication ou le métabolisme cellulaire, en particulier des cellules vivantes de l'épiderme, pour prévenir ou lutter contre l'atrophie de l'épiderme et redonner ainsi à la peau un aspect jeune.

La DHEA ou déhydroépiandrostérone, également connue comme 3-beta-hydroxyandrosterone-5-en-17-one ou dehydroisoandrosterone ou trans-dehydroandrosterone ou prasterone, est un stéroïde naturel produit essentiellement par les glandes corticosurrénales.

La DHEA exogène, administrée par voie topique ou orale, est connue pour sa capacité à promouvoir la kératinisation de l'épiderme (JP-07196467) et à traiter les peaux sèches en augmentant la production endogène et la sécrétion de sébum et en renforçant ainsi l'effet barrière de la peau (US-4,496,556). Il a également été décrit dans le brevet US-5,843,932 l'utilisation de la DHEA pour remédier à l'atrophie du derme par inhibition de la perte de collagène et de tissu conjonctif. Enfin, la Demanderesse a mis en évidence la capacité de la DHEA à lutter contre l'aspect papyracé de la peau (FR-2803513), à moduler la pigmentation de la peau et des cheveux (EP-1092423) et à lutter contre l'atrophie de l'épiderme. Ces propriétés de la DHEA en font un candidat de choix comme actif anti-âge.

Mais la DHEA présente la difficulté d'être très faiblement soluble dans les solvants cosmétiques ou pharmaceutiques couramment utilisés tels que l'eau, les huiles polaires ou apolaires.

Il est en effet connu que la DHEA ne se solubilise que difficilement dans les milieux aqueux, ce qui limite sa formulation dans des compositions cosmétiques ou dermatologiques appliquées par voie topique ou orale. Elle a ainsi tendance à recristalliser.

La DHEA présente en effet plusieurs formes polymorphiques dont le type et la répartition peuvent être mal contrôlés, car dépendants de conditions environnementales et de la manière de préparer le principe actif (Chang et al, J. Pharm. Sci. 84 :1169-1179 (1995)). Il existe entre trois et cinq formes polymorphiques anhydres et au moins trois

formes hydratées. Ces formes polymorphiques ne peuvent être distinguées que par des techniques analytiques telles que diffraction aux rayons X, spectroscopie infrarouge et DSC (differential scanning calorimetry) (WO-00/54763). Selon la source d'approvisionnement en DHEA, il peut y avoir une répartition polymorphique variable de la matière première, ce qui peut potentiellement entraîner des variations significatives de la biodisponibilité thérapeutique et de l'efficacité.

Il s'ensuit une perte d'efficacité et une incertitude quant à la dose de DHEA plus ou moins importante présente dans ces compositions, selon le degré de recristallisation, ce qui va à l'encontre de l'objectif recherché. En outre, cette recristallisation peut modifier la stabilité globale de ces compositions ainsi que leur aspect, ce qui peut détourner l'utilisateur de celles-ci.

Par ailleurs, la maîtrise de la stabilité d'une dispersion particulaire peut s'avérer difficile à réaliser.

Dans le tableau I figurent les différents exemples montrant la faible solubilité de la DHEA dans une phase lipophile :

Tableau I

| NOM INCI | Solubilité (%w/w) |
|---|---|
| Caprylic / capric triglycerides | 1.77% |
| Huile de sésame | 1.40% |
| Isopropyl palmitate | 1.37% |
| Mineral oil | 1.00% |
| Octyl palmitate | 1.00% |
| Cetearyl isononanoate | 0.83% |
| Dimethicone | 0.17% |
| Squalane | 0.10% |
| Cyclomethicone | 0.04% |

Ces solubilités maximales de la DHEA ont été mesurées après 12h d'agitation barreau aimanté, à température ambiante, avec un excès de principe actif dans l'excipient à analyser. La suspension est ensuite filtrée (1.2μm) puis le filtrat est dosé en HPLC.

Au-delà de la concentration mesurée, il y a recristallisation de la DHEA à température ambiante.

Malgré cela, la forme galénique la plus couramment utilisée aujourd'hui est l'émulsion huile dans eau dans laquelle la DHEA est incluse dans la phase lipophile. Mais cette solution reste peu satisfaisante car pour répondre à un objectif de concentration en actif ayant une efficacité thérapeutique quantifiable, il faudrait des concentrations en huiles solvantes très élevées, conduisant à des produits sans aucun doute peu agréables à utiliser ; tout en étant limités en concentration de DHEA.

L'élaboration d'une émulsion inverse (par émulsion inverse, on entend une émulsion de type : phase hydrophile dispersée dans phase lipophile) comme alternative n'était pas évidente pour l'homme du métier compte tenu des difficultés connues de solubilité de la DHEA dans l'eau.

L'utilisation d'autres solubilisants hydrophiles comme le propylène glycol n'état également pas naturelle pour l'homme du métier compte tenu que les fortes concentrations nécessaires n'étaient pas favorables à une bonne stabilité et un toucher cosmétique acceptable.

L'obtention d'une bonne tolérance avec des solubilisants comme le propylène glycol n'était également pas évidente car il avait été montré chez l'homme des phénomènes d'intolérance cutanée, par exemple chez l'homme sain (Motoyoshi et al ,Cosmet and toiletries, 99, 83-89, 1984) où le propylène glycol apparaissait comme irritant à fortes concentrations mais seulement sous occlusion.

Enfin une utilisation d'une composition de type eau dans huile avait été évoquée dans l'art antérieur. Ainsi, FR-2777194 décrit une composition cosmétique ou dermatologique de type eau-dans huile contenant 10 à 50 % d'un hydrocarbure liquide saturé ramifié en C20 à C40 ou d'un mélange de tels hydrocarbures, 1 à 47 % d'un phospholipide naturel ou d'un mélange de phospholipides naturels et 50 à 80 % d'eau. Or même si la DHEA est mentionnée de façon générique dans une liste extensive de composés actifs , aucune donnée concrète n'est fournie. Outre l'absence de réalisation effective dans cet art antérieur, l'homme du métier n'était pas enclin à suivre cette voie de formulation compte tenu du fort pourcentage d'eau enseigné dans FR-2777194 (50 à 80%) et de la faible solubilité de la DHEA dans l'eau (solubilité maximum de 0,02 mg/ml), dans laquelle la DHEA précipite très rapidement.

En outre, l'enseignement de cet art antérieur est principalement centré sur l'utilisation de phospholipides et non sur l'obtention de formulations utilisables pour des dérivés complexes comme la DHEA, et/ou ses précurseurs ou

dérivés chimiques et/ou biologiques.

Or les phospholipides présentent une stabilité chimique limitée vis à vis des phénomène d'oxydation ce qui n'encourageait pas l'homme du métier à utiliser ce document dans sa recherche de formulations stables à la base de DHEA ou analogues

Il existait donc un besoin d'une composition permettant de répondre à un ou plusieurs des aspects suivants : disposer d'une bonne stabilité au froid et à la chaleur, en particulier quant au maintien de la taille des globules et à l'absence de déphasage, avoir une bonne résistance vis-à-vis des phénomènes d'oxydation, permettre une bonne stabilité et biodisponibilité de la DHEA et/ou ses précurseurs ou dérivés chimiques et/ou biologiques, présenter une bonne tolérance cutanée. Il est également utile de pouvoir disposer d'une composition autorisant une forte fraction volumique dispersée. Il est par ailleurs utile que la préparation de telles compositions bénéficie d'un mode de préparation avantageux.

Or la Demanderesse a mis au point de façon surprenante une formulation de type glycol dans huile qui permet de s'affranchir des différents problèmes liés aux aspects mentionnés ci -dessus en permettant notamment de disposer d'une bonne stabilité de la composition en tant que telle mais aussi de permettre une bonne stabilité et biodisponibilité de la DHEA et/ou ses précurseurs ou dérivés chimiques et/ou biologiques qu'elle contient. La composition selon l'invention a également l'avantage de présenter une bonne tolérance cutanée et d'autoriser une forte fraction volumique dispersée.

Il a notamment été découvert qu'il est possible d'utiliser la bonne solubilité de la DHEA à des taux élevés dans des glycols hydrophiles (mise en évidence par la demanderesse) pour obtenir une formulation stable tout en évitant la recristallisation du principe actif.

L'invention se rapporte donc à une composition contenant de la DHEA et/ou ses précurseurs ou dérivés chimiques et/ou biologiques, caractérisée en ce que la composition est une émulsion inverse contenant une phase hydrophile dispersée glycolique ou hydroglycolique, une phase continue lipophile et un émulsionnant siliconé de HLB compris entre 2 et 7, et en ce que la DHEA et/ou ses précurseurs ou ses dérivés chimiques et/ou biologiques est solubilisée dans la phase hydrophile glycolique ou hydroglycolique.

Par le terme HLB, on entend le Rapport Hydrophile/Lipophile ou « Hydrophilic/Lipophilic Balance (HLB) » qui correspond à l'équilibre entre la dimension et la force du groupe hydrophile et la dimension et la force du groupe lipophile de l'émulsionnant.

L'invention permet également de s'affranchir des problèmes occasionnés par le polymorphisme de la DHEA et également d'obtenir une bonne biodisponibilité de l'actif dans la peau, la DHEA étant utilisée de façon sous forme solubilisée.

Par forme solubilisée, on entend une dispersion à l'état moléculaire dans un liquide, aucune cristallisation de l'actif n'étant visible à l'oeil nu ni même au microscope optique en polarisation croisée.

La formulation de la DHEA, et/ou ses précurseurs ou dérivés chimiques et/ou biologiques, solubilisés dans une phase glycolique ou hydroglycolique en émulsion inverse permet ainsi, de manière surprenante de s'affranchir des problèmes de recristallisation, par mûrissement (Kabalnov et AL, J.ColloTd and interface Science, 118(1987) 590-597) de cette dernière.

La présente invention consiste donc à faire des émulsions Inverses, contenant une phase hydrophile glycolique ou hydroglycolique, parfaitement stables (taille des globules et viscosité), même à forte fraction volumique dispersée, ne montrant aucune recristallisation significative de la DHEA , et/ou ses précurseurs ou dérivés chimiques et/ou biologiques.

Par précurseurs de la DHEA, on entend ses précurseurs biologiques immédiats ou substrats, ainsi que ses précurseurs chimiques. Des exmples de précurseurs biologiques sont la Δ5-prégnénolone, la 17 α-hydroxy prégnénolone sans que cette liste soit limitative. Des exemples de précurseurs chimiques sont les sapogénines telles que la diosgénine (ou spirost-5-èn-3-beta-ol), l'hécogénine, l'acétate d'hécogénine, le smilagénine et la sarsapogénine, ainsi que les extraits naturels en contenant, en particulier le fenugrec et les extraits de Dioscorées telle que la racine d'igname sauvage ou « Wild Yam ».

Par dérivés de la DHEA, on entend aussi bien ses dérivés métaboliques que ses dérivés chimiques. Comme dérivés métaboliques, on peut citer notamment la 7-α-OH-DHEA, la 7-β-OH-DHEA, la 7-céto- DHEA, la Δ5. -androstène-3,17-diol et la Δ4-androstène-3,17-dione. Des dérivés chimiques de la DHEA convenant à la mise en ouvre de la présente invention sont les dérivés de formule (1) :

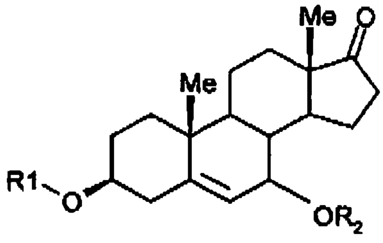

(1)

dans laquelle :

$R_1$ et $R_2$ sont indépendamment choisis parmi :

- un groupe alkyle en $C_1$-$C_{12}$, saturé ou insaturé, linéaire, ramifié ou cyclique pouvant éventuellement contenir un ou plusieurs hétéroatomes, et éventuellement substitué par un ou plusieurs groupes choisis parmi -OR' et/ou -SR' et/ou -COOR' et/ou - NR'R' et/ou halogène et/ou sulfate et/ou phosphate et/ou aryle et/ou hétérocycle, ledit hétérocycle pouvant avantageusement être choisi parmi un indole, une pyrimidine, une pipéridine, une morpholine, un pyrane, un furane, une pipérazine, une pyridine ;
- un groupe alkylcarbonyle, dont la partie alkyle en $C_1$-$C_{24}$ est saturée ou insaturée, linéaire, ramifiée ou cyclique, et éventuellement substituée par un ou plusieurs groupes choisi parmi -OR' et/ou -SR' et/ou -COOR' et/ou -NR'R' et/ou halogène et/ou sulfate et/ou phosphate et/ou aryle et/ou hétérocycle, ledit hétérocycle pouvant avantageusement être choisi parmi un indole, une pyrimidine, une pipéridine, une morpholine, un pyrane, un furane, une pipérazine, une pyridine ;
- un groupe arylcarbonyle, de préférence un phénylcarbonyle, ou un groupe arylalkylcarbonyle, de préférence un benzylcarbonyle, éventuellement substitué par un ou plusieurs groupes -OR' et/ou -SR' et/ou -COOR' et/ou -NR'R' et/ou halogène et/ou aryle et/ou hétérocycle ;
- un groupe O=P(OH)OR' ;
- un groupe $(O)_2SOR'$ ;
- un groupe trialkylsilyle ($SiR'_3$) dans lequel les 3 groupes R' peuvent être identiques ou différents ;
- un groupe carbonyloxyalkyle (R'OCO) ;
- un groupe carbonylaminealkyle (R'NHCO) ;

dans lesquels R' est choisi parmi un atome d'hydrogène, un groupe alkyle en $C_1$-$C_{12}$, de préférence en $C_1$-$C_6$, saturé ou insaturé, linéaire, ramifié ou cyclique pouvant éventuellement contenir un ou plusieurs hétéroatomes, éventuellement fonctionnalisé par un ou plusieurs groupes -OR", -COOR", halogène, -NR"R"; ou par un groupe aryle, de préférence un phényle, éventuellement fonctionnalisé par un ou plusieurs groupes-OR", -COOR", halogène ou -NR"R" ;
R" représentant un atome d'hydrogène, une chaîne alkyle, de préférence en $C_1$-$C_6$, saturée ou insaturée, linéaire, ramifiée ou cyclique,
étant entendu que dans chacun des groupes -NR'R' et -NR"R", les substituants R', respectivement R", sont identiques ou différents.

Parmi les dérivés de formule (1), on peut citer en particulier les diesters de 7-OH-DHEA et plus préférentiellement la 3-O-acetyl-7-benzoyloxydéhydroépiandrostérone qui est notamment disponible auprès de la société GATTEFOSSE sous la dénomination commerciale 3-acetoxy-7-benzoate DHEA.

La composition selon l'invention est de préférence adaptée à une application topique sur la peau, les phanères et/ou les muqueuses . Elle renferme généralement un milieu physiologiquement acceptable et une quantité de composé à base de DHEA suffisante pour obtenir l'effet recherché. La proportion pondérale de DHEA, et/ou ses précurseurs ou dérivés chimiques et/ou biologiques, par rapport au poids total de la composition peut ainsi être comprise entre 0.001% et 20% (poids/poids), par exemple entre 0,1 et 20%, en particulier entre 0.2% et 10%, notamment entre 0,2 et 4 %, par

exemple entre 0,2 et 2%.

Les glycols à considérer dans la présente invention peuvent être définis comme des alkylène ou des poly alkylène glycols . A titre d'exemples non limitatifs, on peut citer les alkylènes et polyalkylènes glycols (C1 à C6) tel que l'éthylène glycol, le polyéthylène glycol (2 à 20 monomères), le propylène glycol, le dipropylène glycol, le butylène glycol, le pentylène glycol, l'hexylène glycol. Ils peuvent être oxyéthylénés ou non (2 à 50 OE). Les préférés, selon l'invention, sont l'hexylène glycol, le propylène glycol et le dipropylène glycol.

Les glycols utilisables selon l'invention auront avantageusement comme paramètre de solubilité un δp inférieur à 10 étant entendu que les 3 paramètres de solubilité de Hansen : δd, δp et δh caractérisent, pour un constituant donné, les énergies correspondant respectivement aux interactions dispersives, polaires et de type liaisons hydrogène existant entre les molécules de ce constituant, δp caractérisant plus particulièrement les forces d'interaction de Debye entre dipôles et étant fonction du nombre d'atomes d'oxygène dans la formule du constituant donné (S. paint Technology, 30, 195, 1967, « The three dimensional solubility parameter-Key to paint component affinities »).

La fraction volumique de la phase hydrophile dispersée dans l'émulsion selon l'invention va de 10 à 90% par rapport au volume total de l'émulsion. Elle peut être exclusivement glycolique ou hydroglycolique, étant entendu que la DHEA, et/ou ses précurseurs ou dérivés chimiques et/ou biologiques, y sont de préférence solubilisés. La proportion volumique de glycols (par rapport au volume total de la phase dispersée) se situe entre 10 et 100%, par exemple entre 30 et 100%, en particulier entre 60 et 100%, et de préférence entre 80 et 100%.

En vue d'une application cosmétique, on utilisera de préférence entre 30 et 50% de glycols (proportion par rapport au volume total de la phase dispersée).

Il est également possible de caractériser un mode de réalisation préféré de l'invention en se rapportant à l'activité en eau ($a_w$) de la phase hydrophile dans la composition selon l'invention.

L'invention se rapporte ainsi également, de façon particulière, à une compositiontelle que définie précédemment , caractérisée en ce que l'activité en eau $a_w$ de la phase hydrophile est inférieure à 0,85.

L'activité en eau $a_w$ d'un milieu contenant de l'eau est le rapport de la pression de vapeur d'eau du produit « $P_{H2O}$ produit » et de la pression de vapeur de l'eau pure « $P_{H2O}$ pur » à la même température. Elle peut être exprimée aussi comme le rapport du nombre de molécules d'eau « $N_{H2O}$ » sur le nombre de molécules totales « $N_{H2O}$ + $N_{corps\ dissous}$ », qui tient compte de celles des corps dissous «$N_{corps\ dissous}$ ».

Elle est donnée par les formules suivantes :

$$a_w = \frac{P_{H2O}\ \text{produit}}{P_{H2O}\ \text{pur}} = \frac{N_{H2O}}{N_{H2O} + N_{corps\ dissous}}$$

On peut utiliser différentes méthodes pour mesurer l'activité en eau $a_w$. La plus courante est la méthode manométrique par laquelle on mesure directement la pression de vapeur.

De manière classique, une composition cosmétique ou dermatologique a une activité en eau située autour de 0,95 à 0,99. Une activité en eau inférieure à 0,85 représente une diminution notable.

Les émulsionnants (ou tensio-actifs ou surfactants) sont des substances naturelles ou synthétiques formées d'une partie hydrophile ou polaire et d'une partie lipophile ou apolaire. Ce sont des molécules amphiphiles puisqu'elles ont une double polarité. Les émulsionnants sont caractérisés par leur HLB ; si le HLB est élevé, la fraction hydrophile est prédominante, si le HLB est faible, la partie lipophile prédomine.

Parmi ces émulsionnants, on inclut de façon préférée les émulsionnants polymériques qui se caractérisent par une masse molaire élevée et une structure non linéaire qui permet un ancrage plus important à l'interface eau/huile que celui obtenu avec les émulsionnants de type monomère.

Les émulsionnants qu'il est possible d'utiliser selon l'invention, seuls ou en mélange, sont ceux permettant de faire des émulsions inverses et ayant une HLB inférieure à 7.

D'une façon générale les émulsionnants préférés sont les organopolysiloxanes tels que :

- E1) Poly Alkyl méthicone Copolyols (Poly alkyl méthylsiloxane oxyalkylénés éventuellement réticulés) contenant :

    des chaînes Alkyle de C6 à C20, saturées ou non, linéaires ou ramifiées
    un motif Polyoxyéthyléné de 1 à 50 OE (Oxyde d'Ethylene) et/ou
    un motif Polyoxypropyléné de 1 à 50 OP (Oxyde de Propylène)

- E2) Poly Alkyl diméthyl méthylsiloxane oxyalkylénés contenant:

  des chaînes alkyle de C6 à C20, saturées ou non, linéaires ou ramifiées
  un motif Polyoxyéthyléné de 1 à 50 OE
  et/ou
  un motif Polyoxypropyléné de 1 à 50 OP

Les organopolysiloxanes de la composition de l'invention contiennent notamment un ou plusieurs groupements oxyalkylénés et en particulier oxyéthylénés (OE), par exemple de 1 à 40 motifs oxyalkylénés, de préférence de 1 à 20, mieux de 10 à 20, de façon plus préférée de 12 à 20 et encore mieux de 12 à 18 motifs oxyalkylénés, pouvant former des chaînes polyoxyalkylènes et notamment polyoxyéthylènes. Ces groupements peuvent être pendants ou en bout de chaîne. Les atomes de silicium portant ces groupements sont avantageusement au nombre d'environ 1 à 10et mieux de 1 à 6. La structure siliconée formant le squelette polymérique de l'organopolysiloxane à groupement(s) oxyalkyléné(s) est avantageusement une structure polydiméthylsiloxane (PDMS) dont éventuellement une partie des groupes méthyle est substituée par des groupements alkyle en C2 à C30 et de préférence en C8 à C24, et mieux de C10 à C20 ou phényle, soit en bout de chaîne soit pendants.

Avantageusement, on utilisera donc comme émulsionnants de type E1 ou E2 les émulsionnants siliconés comme les alkyldiméthicone copolyols tels que l'Abil EM-90, ou le mélange de diméthicone copolyol et cyclométhicone, vendu par la société Dow Coming sous la dénomination 3225C Formulation Aid, le laurylmethicone copolyol vendu sous le nom d'Emulsifier 10 par Dow Coming, ou des mélanges à base d'un polymère siliconé tel que le cetyl dimethicone copolyol avec du polyglyceryl-4 isostéarate et de l'hexyl laurate vendu sous le nom d'Abil WE09 par la société Goldschmidt, l'Abil EM 97 de Goldschmidt (Dimethicone copolyol & cyclomethicone), le Wacker SPG 128 VP de Wacker (cyclomethicone et octyldimethicone methoxy glycosyl), ou encore le Silwax WD-IS (Dimethicone copolyol iso-stearate)

E3) les mono ou polyalkylesters siloxanes, par exemple le Silwax S de Lambent (Dimethiconol stearate),

On utilisera les émulsionnants siliconés de HLB compris entre 2 et 7, préférentiellement un émulsionnant E/H siliconé de HLB compris entre 2 et 7, préférentiellement un émulsionnant E/H siliconé polymérique de HLB compris entre 2 et 7.

L'émulsion inverse de l'invention peut être en variante réalisée et stabilisée de façon avantageuse avec les émulsionnants ou les associations à caractère émulsionnant suivantes.

1) l'association d'un organopolysiloxane élastomère réticulé oxyalkyléné et d'un polymère poly(acide 2-acrylamido 2-méthylpropane sulfonique) réticulé et au moins partiellement neutralisé.

En particulier, l'organopolysiloxane à groupement(s) oxyalkyléné(s) peut comporter un ou plusieurs squqelette(s) siliconé(s) relié(s) entre eux par un ou plusieurs groupements oxyalkylénés et de préférence oxyéthylénés tels que définis précédemment, ou par un ou plusieurs groupements alkylénés, le nombre de groupement alkyléné allant de 1 à 30 et de préférence de 1 à 20. De préférence, Il comporte au moins deux squelettes polymériques liés entre eux.

Avantageusement, le ou les squelettes siliconés des organopolysiloxanes de la composition selon l'invention comportent de 26 à 80 atomes de silicium. Les organopolysiloxanes élastomères utilisés dans la composition conforme à l'invention sont partiellement ou totalement réticulés et de structure tridimensionnelle. Inclus dans une phase lipophile, ils se transforment, selon le taux de phase lipophile utilisé, d'un produit d'aspect spongieux lorsqu'ils sont utilisés en présence de faibles teneurs en phase lipophile, en un gel homogène en présence de quantités de phase lipophile plus élevées. La gélification de la phase lipophile par ces élastomères peut être totale ou partielle. Ces organopolysiloxanes élastomères peuvent se présenter sous forme de poudre, les particules constituant cette poudre ayant une taille allant généralement de 0,1 à 500 $\mu$m, de préférence de 3 à 200 $\mu$m et mieux de 3 à 50 $\mu$m, et pouvant être sphériques, plates ou amorphes avec, de préférence, une forme sphérique. Ils peuvent aussi se présenter sous forme de gel anhydre contenant l'organopolysiloxane élastomère dispersé dans une phase huileuse. Les organopolysiloxanes de la composition de l'invention sont par exemple celui commercialisé sous la référence KSG 21 par la société Shin Etsu ou le produit de l'exemple 3 (exemple de synthèse) du brevet US-5,412,004.

2) Les alkylpolyglycosides ayant un HLB inférieur à 7, associés à un polydiméthylsiloxane oxyalkyléné. La chaîne alkyle de l'alkylpolyglycoside comporte de préférence de 14 à 22 atomes de carbone et peut être notamment une chaîne linéaire insaturée ou une chaîne ramifiée, et plus particulièrement la chaîne oléyle ou isostéaryle. Les alkylpolyglycosides utilisés selon la présente invention peuvent être plus particulièrement représentés par la formule générale (I) suivante :

$$R\text{-}O\text{-}(G)x \qquad (I)$$

dans laquelle R représente un radical alkyle linéaire insaturé ou un radical alkyle ramifié, comportant de 14 à 24 atomes de carbone, G représente un sucre réduit comportant de 5 à 6 atomes de carbone, et x désigne une valeur allant de 1 à 15. Des alkylpolyglycosides préférés selon la présente invention sont des composés de formule (I) dans laquelle R désigne plus particulièrement un radical alkyle comportant de 16 à 22 atomes de carbone, G désigne le glucose, le fructose ou le galactose, x est une valeur allant 1 à 4 et plus particulièrement de 1 à 2. Selon l'invention, dans la formule (1). R est un radical alkyle linéaire insaturé (c'est-à-dire radical alkylène) ou un radical alkyle ramifié. Le radical alkyle insaturé peut comprendre une ou plusieurs insaturations éthyléniques, et en particulier une ou deux insaturations éthyléniques. Selon un mode préféré de réalisation de l'invention, le radical R comporte 18 atomes de carbone et désigne notamment un radical oléyle (radical insaturé en C18) ou isostéaryle (radical saturé en C18), G désigne le glucose et x est une valeur allant de 1 à 2. L'alkylpolyglycoside utilisé dans l'émulsion de l'invention est de préférence choisi dans le groupe comprenant l'isostéaryl-glucoside, l'oléyl-glucoside et leurs mélanges. Les polydiméthylsiloxanes oxyalkylénés considérés sont ceux décrit paragraphe E1 ci-dessus.

La composition selon l'invention contiendra notamment, exprimé en pourcentage en poids, de 0,5 à 8% d'émulsionnant, par exemple de 0,5 à 5 %, de préférence entre 3 et 5 %, par rapport au poids total de la composition.

Par ailleurs, de façon avantageuse, pour améliorer la stabilité de la dispersion, il est possible de compléter le ou les émulsionnants principaux décrits plus haut par un ou plusieurs co-émulsionnants ayant une HLB supérieure à 6. Le rapport (co-émulsionnant / émulsionnant) sera avantageusement inférieur à 1,5 et de préférence inférieur à 0,75.

A titre d'exemple, on peut citer les :

- Alkyl ou poly alkyl esters de sorbitan polyoxyéthylénés ou non avec entre 1 et 5 chaînes alkyles entre C10 et C20 saturées ou non, ramifiées ou non, et avec de 0 à 40 OE (par exemple : monolaurate de sorbitane 20OE ou monooléate de sorbitan 200 OE (Tween 80 de Uniqema))
- Alkyl ou poly alkyl éthers ou esters polyoxyéthylénés avec entre 1 et 5 chaînes alkyles entre C10 et C20 saturées ou non, ramifiées ou non et avec de 0 à 40 OE ( cétéareth-20 (Eumulgin B2 de Cognis), ou le stéareth (Brij 78) 20 OE)
- Les Alkyl ou poly alkyl mono ou polyglucosides éthoxylés et estérifiés avec entre 1 et 5 chaînes alkyles entre C6 et C20 saturées ou non, ramifiées ou non et de 1 à 10 motifs de glucose (par exemple, le PEG-20 methylglucose sesqui stéarate (Glucamate SSE-20 de Amerchol)).
- Les Alkyl ou poly alkyl esters ou éthers de polyglycérol avec entre 1 et 5 chaînes alkyles entre C10 et C20 saturées ou non, ramifiées ou non et de 1 à 8 motifs glycérol (par exemple le polyglycéryl 4 - isostéarate ou le PEG-8 stéarate (Myrj 45))

Enfin, il est possible d'ajouter de façon avantageuse dans la phase dispersée, de 0 à 10% en poids,par rapport au poids total de la formulation, d'un co-solvant de la DHEA ayant une température d'évaporation inférieure à 100°C, de préférence des alcools hydrophiles linéaires ou ramifiés de C1 à C4 , comme l'éthanol et l'isopropanol.

De façon intéressante, la préparation de l'émulsion selon l'invention s'est avérée ne nécessiter que peu d'énergie mécanique ou thermique par rapport aux préparations d'autres émulsions inverses déjà connues.

De façon connue, la composition de l'invention peut contenir également les adjuvants habituels dans les domaines cosmétique et dermatologique, tels que les gélifiants hydrophiles ou lipophiles, les humectants comme la glycérine et le sorbitol, les actifs hydrophiles ou lipophiles, les épaississants de phase grasse, les conservateurs les antioxydants, les électrolytes, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase lipophile ou dans la phase hydrophile Ces adjuvants, ainsi que leurs concentrations, doivent être tels qu'ils ne nuisent pas aux propriétés cosmétiques ou dermatologiques de la DHEA et/ou ses précurseurs ou dérivés chimiques et/ou biologiques, dans la composition selon l'invention.

Comme matières grasses utilisables pour la phase lipophile continue dans les émulsions selon l'invention, on peut utiliser les huiles, et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras tels que l'alcool cétylique, des acides gras, des cires et des gommes et en particulier les gommes de silicone.

De façon préférée, on utilise des matières grasses non oxydables pour les huiles de la phase lipophile continue, qui sont préférentiellement choisies parmi celles de type siliconé, celles de type ester ou celles de type minéral.

D'une façon préférée, la phase lipophile est non solvante de la. DHEA.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Comme actifs, on peut utiliser notamment les isoflavonoïdes, les inhibiteurs de métalloprotéinase, les caraténoïdes, les composés anti-glycation, les inhibiteurs de NO-synthase, les vitamines, les agents desquamants, les composés augmentant la synthèse des glycosaminoglycanes, les composés anti-irritants, les composés diminuant l'irritation d'origine neurogène, les composés myorelaxants et les dépigmentants.

La composition selon l'invention a un toucher cosmétiquement acceptable, une bonne tolérance cutanée, une stabilité (par stabilité, on entend la stabilité physique, c'est à dire absence de déphasage et maintien de la taille des globules et non recristallisation de l'actif) au froid (à 4° C) et à la chaleur (45°C) sur une longue durée, par exemple sur 2 mois, avec une viscosité stable.

L'invention s'étend également à une composition qui est une émulsion triple de type phase hydrophile/phase lipophile/phase hydrophile comportant une phase hydrophile externe, et une phase lipophile constituant avec une phase hydrophile interne une émulsion inverse (dite émulsion inverse primaire dans le cadre de cette émulsion triple) selon l'invention comprenant un émulsionnant siliconé.

De façon avantageuse, la présente invention se rapporte à une émulsion triple de type phase hydrophile/phase lipophile/phase hydrophile où la phase hydrophile interne de l'émulsion triple a une valeur d'activité en eau inférieure ou égale à 0,85, notamment en vue d'améliorer la stabilité de l'actif présent dans la phase hydrophile interne.

Selon un mode particulier de réalisation de l'invention, la valeur d'activité en eau inférieure ou égale à 0,85 est obtenue par incorporation d'une quantité efficace de glycol. On entend par quantité efficace une quantité suffisante de polyol pour obtenir une faible valeur d'activité en eau, c'est-à-dire une valeur d'activité en eau inférieure ou égale à 0,85.

Selon un mode particulier de réalisation de l'invention, l'émulsion inverse primaire constitue de 20 à 35 % et plus particulièrement environ 25 % en poids de l'émulsion triple.

L'émulsion triple est préparée de manière classique par préparation de l'émulsion primaire et incorporation d'une quantité déterminée de l'émulsion primaire dans la phase hydrophile externe.

L'invention s'étend aussi à une émulsion triple de type phase hydrophile/phase lipophile/phase hydrophile comportant une phase hydrophile externe, une phase lipophile constituant avec une phase hydrophile interne une émulsion inverse (dite émulsion inverse primaire dans le cadre de cette émulsion triple) selon l'invention comprenant un émulsionnant siliconé et comportant une phase hydrophile externe gélifiée contenant :

1) au moins un copolymère émulsionnant constitué d'une fraction majoritaire d'un monomère acide carboxylique monooléfiniquement Insaturé en $C_3$-$C_8$ ou de son anhydride et d'une fraction minoritaire de monomère ester gras d'acide acrylique, et
2) au moins un poly-(acide acrylamidométhyl propane sulfonique) réticulé.

Par ailleurs, selon un mode préféré de réalisation de l'invention, la phase lipophile de l'émulsion triple selon l'invention contient au moins une huile de silicone et/ou un émulsionnant siliconé.

Les copolymères émulsionnants utilisables dans l'émulsion triple selon la présente invention sont préparés en polymérisant une quantité prépondérante d'un monomère carboxylique monooléfiniquement insaturé ou de son anhydride, à une quantité plus faible de monomère ester acrylique à chaîne grasse. On entend par chaîne grasse un radical alkyle linéaire ou ramifiée, comportant de 8 à 30 atomes de carbone.

La quantité de monomère carboxylique ou de son anhydride va, de préférence, de 80 à 98 % en poids et plus particulièrement de 90 à 98 % en poids, tandis que le monomère ester acrylique est présent dans des quantités allant de 2 à 20 % en poids et plus particulièrement de 1 à 10 % en poids, les pourcentages étant calculés par rapport au poids des deux monomères.

Les monomères carboxyliques préférentiels sont choisis parmi ceux répondant à la formule (I) suivante :

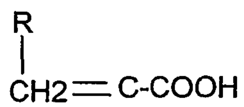 (I)

où R désigne l'hydrogène, un halogène, un groupe hydroxyle, un groupe lactone, un groupe lactame, un groupe cyanogène (-C=N), un groupe alkyle monovalent, un groupe aryle, un groupe alkylaryle, un groupe aralkyle ou un groupe cycloaliphatique.

Les monomères carboxyliques particulièrement préférés sont choisis parmi l'acide acrylique, l'acide méthacrylique ou leurs mélanges.

Les monomères esters acryliques à chaîne grasse sont préférentiellement choisis parmi ceux répondant à la formule (II) suivante :

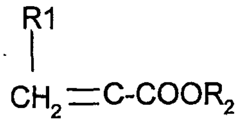 (II)

où $R_1$ est choisi dans le groupe formé par l'hydrogène, le radical méthyle et le radical éthyle, et $R_2$ est un radical alkyle en $C_8$-$C_{30}$.

Les monomères esters particulièrement préférés sont ceux pour lesquels $R_1$ est l'hydrogène ou un radical méthyle et $R_2$ est un radical alkyle en $C_{10}$-$C_{22}$.

Les copolymères émulsionnants peuvent être éventuellement réticulés à l'aide d'un agent réticulant utilisé en une quantité allant de 0,1 à 4 %, de préférence de 0,2 à 10 % en poids par rapport au poids total de monomères carboxyliques et de monomères esters acryliques. L'agent réticulant est choisi parmi les monomères polymérisables contenant un groupe $CH_2$=C- polymérisable et au moins un autre groupe polymérisable, dont les liaisons insaturées ne sont pas conjuguées l'un par rapport à l'autre.

Les copolymères émulsionnants de l'invention sont décrits dans la demande EP-A-0268164 et sont obtenus selon les méthodes de préparation décrites dans ce même document.

Les copolymères émulsionnants particulièrement préférés sont ceux présentant une viscosité mesurée au viscosimètre BROOKFIELD dans une solution d'eau à 2% et à 25° C, inférieure ou égale à 5000 cps (5 Pa.s) et plus préférentiellement de l'ordre d'environ 3000 cps (3 Pa.s).

On utilise plus particulièrement un copolymère acrylate/$C_{10}$-$C_{30}$-alkylacrylate et notamment celui vendu sous le nom PEMULEN TR 1 par la Société GOODRICH.

Le copolymère émulsionnant est utilisé dans l'émulsion triple selon l'invention en une concentration allant, par exemple, de 0,05 à 3 % et de préférence de 0,1 à 1 %, et mieux de 0,2 à 0,6 % du poids total de l'émulsion.

L'invention couvre également l'utilisation de la nouvelle émulsion inverse telle que décrit précédemment en cosmétique et en dermatologie.

La composition trouve ainsi une application en cosmétique notamment pour traiter et/ou protéger la peau, les muqueuses ou les fibres kératiniques, c'est à dire les cheveux et les cils.

La composition selon l'invention trouve également une application dans la prévention et/ou le traitement des signes du vieillissement cutané chronologique ou actinique ainsi que dans le traitement de certaines pathologies.

La présente invention concerne donc également l'utilisation cosmétique de la composition mentionnée ci-dessus pour prévenir et/ou traiter le vieillissement chronologique ou actinique, en particulier:

- pour prévenir ou réduire l'aspect papyracé de la peau, et/ou
- pour améliorer l'homogénéité de la couleur de la peau et/ou pour blanchir la peau et/ou raviver l'éclat du teint, et/ou
- pour traiter les rides et ridules, et/ou
- pour lutter contre le relâchement cutané, et/ou
- pour lutter contre ou prévenir l'atrophie de la peau et des muqueuses,
- pour lutter contre la sécheresse de la peau.

Elle concerne aussi l'utilisation de cette composition pour le traitement cosmétique du cuir chevelu, en particulier pour prévenir ou traiter la canitie.

La présente invention concerne également l'utilisation cosmétique de la composition selon l'invention pour atténuer les taches pigmentaires

L'invention s'étend par ailleurs à l'utilisation d'une composition selon, l'invention pour fabriquer une préparation pharmaceutique, notamment pour fabriquer une préparation pharmaceutique destinée à prévenir ou à traiter l'atrophie de la peau ou des muqueuses, en particulier destinée à prévenir ou à traiter l'atrophie vulvaire ou vaginale.

L'invention couvre également les préparations pharmaceutiques et les médicaments obtenus à partir des compositions selon l'invention.

L'invention sera maintenant illustrée par les exemples non limitatifs suivants. Dans ces exemples, les quantités sont indiquées en pourcentage pondéral, sauf indication contraire.

**EXEMPLES**

Dans les compositions ci-après (Exemples 1 à 6) les proportions des différents constituants sont exprimées en pourcentages en poids. Elles sont préparées de la façon suivante :

Préparation de la Phase B1:

La DHEA est solubilisée dans le Propylène Glycol.

Préparation de la Phase B2:

L'électrolyte (MgS04 ou NaCl) est dissout dans l'eau.
Additionner les Phases B2 et B3 à la phase B1 et chauffer à 50°C.

Préparation de la Phase A :

Les constituants hydrophobes sont mélangés et chauffés à 50°C.
La Phase B est incorporée à la Phase A sous agitation mécanique modérée.
La DHEA utilisable selon l'invention est par exemple disponible auprès de la société AKZO NOBEL

Exemple 1:

| | |
|---|---|
| Phase A : | |
| Emulsifier 10 (lauryl methicone copolyol) | 5.00 % |
| Cyclomethicone | 15.00 % |
| Huile de paraffine légère | 15.00 % |
| Alcool cétostéarylique | 3.00 % |
| Phase B1 : | |
| Propylène Glycol | 19.00 % |
| Dipropylène Glycol | 32.00 % |
| Glycérine | 10.00 % |
| DHEA | 1.00 % |

Exemple 2:

| | |
|---|---|
| Phase A : | |
| Emulsifier 10 (lauryl methicone copolyol) | 3.00 % |
| Cyclomethicone | 10.00 % |
| Huile de Paraffine | 10.00 % |
| Ceteareth-20 | 1.00 % |
| Phase B1: | |
| Propylène Glycol | 75.00 % |
| DHEA | 1.00 % |

Exemple 3 :

| | |
|---|---|
| Phase A : | |
| Emulsifier 10 (lauryl methicone copolyol) | 3.00 % |
| Cyclométhicone | 10.00 % |
| Cétéaryl isononanoate | 7.00 % |
| Huile de Paraffine | 3.00 % |
| Cétéareth-20 | 1.00 % |

(suite)

| | |
|---|---|
| Phase B1: | |
| Propylène Glycol | 58.00 % |
| DHEA | 2.00 % |
| Phase B2 : | |
| Eau | 10.00 % |
| MgS04 | 1.00 % |
| Phase B3: | |
| Ethanol | 5.00 % |

Exemple 4 :

| | |
|---|---|
| Phase A : | |
| Emulsifier 10 (lauryl methicone copolyol) | 3.00 % |
| Cyclomethicone | 15.00 % |
| C12-C15 Alkyl Benzoate | 15.00 % |
| Phase B1 : | |
| Propylène Glycol | 56.00 % |
| DHEA | 1.00 % |
| Phase B2 : | |
| Eau | 10.00 % |

Exemple 5 :

| | |
|---|---|
| Phase A : | |
| Diméthicone copolyol et Cyclométhicone | 3.00 % |
| Cyclométhicone | 10.00 % |
| Cétéaryl Isononanoate | 7.00 % |
| Huile de Paraffine | 3.00 % |
| Cétéareth-20 | 1.00 % |
| Stéarate de Zinc | 1.00 % |
| Phase B1 : | |
| Propylène Glycol | 48.00 % |
| DHEA | 1.00 % |
| Phase B2 : 82: | |
| Eau | 20.00 % |
| NaCl | 1.00 % |
| Phase B3: | |
| Ethanol Rectapur | 5.00 % |

Exemple 6:

| | |
|---|---|
| Phase A : | |
| Alkylméthicone copolyol | 3.00 % |
| Cyclomethicone | 10.00 % |
| CetearylIsononanoate | 7.00 % |

(suite)

| | |
|---|---|
| Phase A : | |
| Huile de Paraffine | 3.00 % |
| Ceteareth-20 | 1.00 % |
| Phase B1 : | |
| Propylène Glycol | 49.30 % |
| DHEA | 1.00 % |
| Phase B2 : | |
| Eau | 20.00 % |
| MgSO4 | 0.70 % |
| Phase B3: | |
| Ethanol | 5.00 % |

Données Rhéologiques de la formule de l'exemple 6:

On a utilisé un rhéomètre HAAKE VT 510 avec un mobile de mesure SVDIN. Les rhéogrammes ont été réalisés à 25°C en faisant varier la vitesse de cisaillement avec le temps, et en mesurant la contrainte. Les résultats obtenus sont rassemblés dans le tableau ci-dessous :

| | Seuil d'écoulement ($\tau$ o) en Pa à température ambiante | Seuil d'écoulement ($\tau$ o) en Pa à 45°C |
|---|---|---|
| T = 0 | 61 | / |
| T=1 mois | 52 | Non réalisé |
| T=2 mois | 52 | 48 |

Par seuil d'écoulement ($\tau$ o), on entend la force nécessaire (contrainte de cisaillement minimum) pour vaincre les forces de cohésion du type Van der Waals et provoquer l'écoulement. Ces données indiquent que la viscosité du produit est stable 2 mois à température ambiante et à 45°C.

Exemple 7 :

| | |
|---|---|
| Phase A : | |
| Alkylmethicone copolyol | 3.00 % |
| Cyclomethicone . | 6.00 % |
| Cetearyl isononaoate | 7.00 % |
| Huile de Paraffine | 3.00 % |
| Ceteareth-20 | 1.00 % |
| B.H.T | 0.10 % |
| Phase B: | |
| Propylene Glycol | 58.40 % |
| DHEA | 1.50 % |
| Phase C: | |
| Eau | 14.00 % |
| Electrolytes | 1.00 % |
| Ethanol | 5.00 % |

Préparation de la Phase B:

La DHEA est solubilisée dans le Propylène Glycol à 55°C.

Préparation de la Phase A :

Les constituants hydrophobes sont mélangés et chauffés à 50°C.
La Phase B est incorporée à la Phase A sous agitation mécanique modérée à 50°C.

Préparation de la Phase C:

L'électrolyte est dissout dans l'eau. Puis l'éthanol est incorporé.
Cette phase est introduite à température ambiante dans l'émulsion sous agitation modérée.

Données Rhéologiques de la formule de l'exemple 7:

De la même façon que pour l'exemple 6 les données suivantes sont obtenues :

| | Seuil d'écoulement ($\tau 0$) en Pa à température ambiante |
|---|---|
| T = 0 | 74 |
| T = 3 mois | 69 |

Données sur la stabilité chimique de la DHEA dans le produit fini :

| | Quantité de DHEA dans le produit fini en % |
|---|---|
| T = 0 | 102.0 |
| T = 1 mois à 55°C | 100.8 |
| T = 2 mois à 55°C | 99.9 |
| T = 3 mois à température ambiante | 100.7 |
| T = 3 mois à 55°C | 98.8 |

Exemple 8 :

| Phase A | |
|---|---|
| Mélange à base de cétyl dimethicone copolyol avec du polyglycéryl-4 isostéarate et de l'hexyl laurate (Abil WE 09 de Goldschmidt) | 3.5 % |
| Polyisobutène hydrogéné | 16.5 % |
| Diméthicone (PM 250000) | 4 % |
| Phase B | |
| Dipropylène glycol | 35 % |
| Propylène glycol | 24 % |
| DHEA | 1 % |
| Eau distillée | 15 % |

La DHEA a été préalablement solubilisée dans le dipropylène glycol et les autres constituants de la phase B ont ensuite été ajoutés pour constituer celle ci.
La phase B est introduite dans la phase A sous agitation type pale ou rotor stator. L'émulsion est faite à une température inférieure à 40°C. On obtient alors une composition légèrement visqueuse, stable, ne montrant pas de recristallisation de la DHEA après au moins 15j à 4°C

Exemples 9 à 16 : Compositions contenant de la 7-α-OH-DHEA

D'une façon analogue à celle décrite précédemment, il est possible de préparer les compositions correspondantes aux exemples 1 à 8 mais où on utilise à la place de la DHEA son dérivé métabolique : la 7-α-OH-DHEA.

Exemples 17 à 24 : Compositions contenant de la 7-céto-DHEA

D'une façon analogue à celle décrite précédemment, il est possible de préparer les compositions correspondantes aux exemples 1 à 8 mais où on utilise à la place de la DHEA son dérivé métabolique : la 7-céto-DHEA.

Exemple 25:

Etude de la libération et la pénétration de la DHEA dans une formulation selon l'invention.

Protocole :

La libération pénétration in vitro de la DHEA et/ou ses précurseurs ou dérivés chimiques et/ou biologiques dans des compostions selon l'invention peut être évaluée sur de la peau humaine totale.

La formulation testée est appliquée pendant 16 heures sur des cellules de diffusion en verre (3 mL; 1 $cm^2$). La peau totale non dermatomée a été utilisée. La peau a été fixée sur une cellule de diffusion, le derme étant en contact avec une solution saline physiologique supplémentée à 0.25 % (p/p) par un émulsionnant (liquide récepteur). Le système a été maintenu en mode statique (pas de renouvellement du liquide récepteur en fonction du temps).

Des plasties abdominales et/ou mammaires provenant d'opérations de chirurgie esthétique ont été utilisées. La formulation est appliquée sur ces trois prélèvements de peau différents à raison de 10 mg de formulation par $cm^2$. Les applications ont été réalisées sans occlusion. Les applications étant réalisées en duplicate, les formulations ont donc été appliquées 6 fois au total.

A la fin du temps d'application, pour chaque cellule de diffusion, l'excès de surface est enlevé, le liquide récepteur et la peau sont prélevés. L'épiderme (stratum corneum inclus) est séparé du derme. Pour chaque formulation testée, un bilan total du principe actif est calculé en tenant compte de l'excès et des quantités retrouvées dans la peau et dans le liquide récepteur. Les concentrations de principe actif sont déterminées grâce à un dosage HPLC avec à une détection APCI/MS/MS (limite de quantification: 10 $ng.mL^{-1}$).

Résultat : il est notamment montrée que la formulation selon l'invention permet de retrouver de façon importante dans la peau des quantités de DHEA.

Exemple 26 : Mesure de la Tolérance cutanée

Protocole :

Des applications topiques des compositions selon l'invention sont répétées durant 2 semaines sur l'oreille droite des souris face interne (excepté durant le week end).

Les formulations sont mises dans des tubes, ceux-ci étant stockés à température ambiante. Chaque tube est identifié par une étiquette où figureront le numéro d'étude, le nom du produit, le numéro de formulation, la dose et la date de péremption.

Les animaux utilisés sont des souris Balb/C Albinos Consanguine femelles âgées de 7 - 8 semaines en début d'étude provenant d'IFFA CREDO, France, par lots de 8 souris. Les animaux sont maintenus en acclimatation au moins 5 jours avant le début de l'étude.

Les animaux sont pesés à J1, à la mise en étude et mis en cage individuelle.

20 μL des compositions à tester sont appliquées sur l'oreille droite des souris, face interne, à l'aide d'une multipette (Eppendorf 4780 réglée sur la position 2, munie de son embout Combitip Eppendorf 0.5 mL).

Les traitements se font à raison d'une application par jour, 5 jours par semaine durant 2 semaines, la durée du traitement étant variable en fonction des résultats de l'irritation.

Les animaux sont observés à J1 avant le premier traitement, et tous les deux jours jusqu'à la fin de l'étude.

Les mesures d'épaisseurs d'oreilles sont faites à l'aide d'un oditest et les épaisseurs d'oreilles et les observations cliniques sont notées.

Les moyennes des épaisseurs d'oreilles par lot et les aires sous courbes sont calculées et représentées par des graphes et des histogrammes correspondants.

Les analyses statistiques correspondantes sont faites sur Minitab, les tests utilisés sont Mann-withney et sample-t.

Résultats

La composition selon l'invention testée et son placebo, appliqués dans les mêmes conditions, ne présentent pas de réponse d'irritation significative. Elle est donc considérée comme non irritante.

Exemple 27: Mesure de l'activation de la lipogénèse par les formulations selon l'invention.

L'étude est menée sur des hamsters Syrien femelles. Chez ces animaux, les glandes sébacées situées sur la face interne des oreilles présentent des caractéristiques proches de celles du visage chez l'homme (nombre, structure, composition du sébum).

Protocole:

Les animaux sont maintenus en cages individuelles durant toute la durée de l'étude et ont accès en permanence à l'eau et à la nourriture (Hamster RJ : AURA (IOPS Han). La température des salles est de 22 +/- 2°C avec une hygrométrie de 55 +/-15 %. Les manipulations effectuées sur les animaux sont en accord avec la législation en vigueur sur l'utilisation et la protection des animaux de laboratoire. Les hamsters reçoivent unedose quotidienne des formulations à tester, des placebos, témoins négatifs ou des témoins positifs, par voie topique pendant 10 jours sur la face interne de l'oreille droite. A la fin du traitement et après prélèvement, le cartilage des oreilles droite et gauche est éliminé à l'aide d'un scalpel et on réalise des biopsies de peau d'un diamètre de 8 mm pour les analyses de composition en lipides et d'histologie.

Analyses:

Les différents groupes d'étude sont composés de 10 animaux: 5 animaux sont destinés à l'étude de la composition en lipides des glandes sébacées par chromatographie sur couche mince ; 5 animaux sont destinés à une analyse histologique.

Analyse de la composition en lipides : la peau prélevée sur chaque animal est mise en survie dans des plaques de culture 12 puits contenant du milieu DMEM complémenté en sérum foetal bovin (10%), en présence d'antibiotiques et d'antifongiques. De l'acétate radiomarqué est ajouté aux cultures et celles-ci sont maintenues en incubateur à 37°C pendant une durée de 6 heures. Les biopsies sont alors récupérées, rincées à l'aide de PBS. Les lipides radiomarqués sont extraits dans différents mélanges de solvants selon la procédure de Blye & Dyer et repris dans 400 μl d'un mélange dichlorométhane/ méthanol (2/1). Les échantillons sont alors déposés par groupe de 20 sur des plaques de silices 10x20 pour HPTLC à l'aide d'un robot de dépôt (Camag- ATSIV). Des échantillons de standards sont également déposés en parallèle puis les plaques sont développées dans un système de migration triple. Après carbonisation au sulfate de cuivre, elles sont séchées et mises à exposer durant 16 heures dans des cassettes contenant un film « phosphorimager ». Les différents lipides sont identifiés par comparaison au standard. Les fractions lipidiques sont ensuite analysées et quantifiées à l'aide du logiciel TINA et les résultats sont traités à l'aide du logiciel Excel.

Analyse histologique : les biopsies de peau sont maintenues dans des cassettes pour histologie entre deux tampons de mousse imbibés de paraformaldéhyde 10% puis incluses dans de la paraffine. Elles sont coupées et colorées à l'aide d'un mélange hémalin-phloxine-safran. Les mesures d'épaisseur d'épiderme sont alors réalisées.

Résultats:

Il apparaît que la formulation selon l'invention permet l'induction de la lipogénèse de manière dose-dépendante sur la peau traitée tout en conservant une bonne tolérance cutanée.

**Revendications**

**1.** Composition contenant de la DHEA et/ou ses précurseurs choisis parmi la Δ5-prégnénolone, la 17 α-hydroxy prégnénolone, la diosgénine, l'hécogénine, l'acétate d'hécogénine, le smilagénine et la sarsapogénine, ou ses dérivés chimiques et/ou biologiques choisis parmi la 7-α-OH-DHEA, la 7-β-OH-DHEA, la 7-céto- DHEA, la Δ5-androstène-3,17-diol, la Δ4 -androstène-3,17-dione, et les dérivés de la DHEA de formule (1):

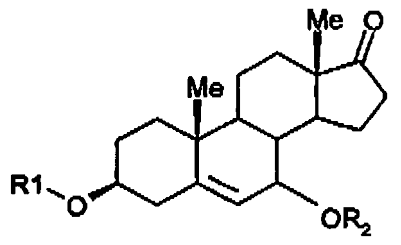

(1)

dans laquelle :

$R_1$ et $R_2$ sont indépendamment choisis parmi :

- un groupe alkyle en $C_1$-$C_{12}$, saturé ou insaturé, linéaire, ramifié ou cyclique pouvant éventuellement contenir un ou plusieurs hétérostomes, et éventuellement substitué par un ou plusieurs groupes choisis parmi -OR’ et/ou -SR’ et/ou -COOR’ et/ou - NR’R’ et/ou halogène et/ou sulfate et/ou phosphate et/ou aryle et/ou hétérocycle, ledit hétérocycle pouvant avantageusement être choisi parmi un indole, une pyrimidine, une pipéridine, une morpholine, un pyrane, un furane, une pipérazine, une pyridine ;
- un groupe alkylcarbonyle, dont la partie alkyle en $C_1$-$C_{24}$ est saturée ou insaturée, linéaire, ramifiée ou cyclique, et éventuellement substituée par un ou plusieurs groupes choisi parmi -OR’ et/ou -SR’ et/ou -COOR’ et/ou -NR’R’ et/ou halogène et/ou sulfate et/ou phosphate et/ou aryle et/ou hétérocycle, ledit hétérocycle pouvant avantageusement être choisi parmi un indole, une pyrimidine, une pipéridine, une morpholine, un pyrane, un furane, une pipérazine, une pyridine ;
- un groupe arylcarbonyle, de préférence un phénylcarbonyle, ou un groupe arylalkylcarbonyle, de préférence un benzylcarbonyle, éventuellement substitué par un ou plusieurs groupes-OR’ et/ou-SR’ et/ou-COOR’ et/ou-NR’R’ et/ou halogène et/ou aryle et/ou hétérocycle ;
- un groupe O=P(OH)OR’ ;
- un groupe $(O)_2SOR’$ ;
- un groupe trialkylsilyle ($SiR’_3$) dans lequel les 3 groupes R’ peuvent être identiques ou différents ;
- un groupe carbonyloxyalkyle (R’OCO) ;
- un groupe carbonylaminealkyle (R’NHCO) ;

dans lesquels R’ est choisi parmi un atome d’hydrogène, un groupe alkyle en $C_1$-$C_{12}$, de préférence en $C_1$-$C_8$, saturé ou insaturé, linéaire, ramifié ou cyclique pouvant éventuellement contenir un ou plusieurs hétéroatomes, éventuellement fonctionnalisé par un ou plusieurs groupes -OR", -COOR", halogène, -NR"R"; ou par un groupe aryle, de préférence un phényle, éventuellement fonctionnalisé par un ou plusieurs groupes-OR", -COOR", halogène ou -NR"R";
R" représentant un atome d’hydrogène, une chaîne alkyle, de préférence en $C_1$-$C_8$, saturée ou insaturée, linéaire, ramifiée ou cyclique,
étant entendu que dans chacun des groupes -NR’R’ et -NR"R", les substituants R’, respectivement R", sont identiques ou différents,
**caractérisée en ce que** la composition est une émulsion inverse contenant une phase hydrophile dispersée glycolique ou hydroglycolique, une phase continue lipophile et un émulsionnant siliconé de HLB compris entre 2 et 7, et **en ce que** la DHEA et/ou ses précurseurs ou ses dérivés chimiques et/ou biologiques est solubilisée dans la phase hydrophile dispersée glycolique ou hydroglycolique.

**2.** Composition selon la revendication 1 **caractérisée en ce que** l’émulsionnant est choisi parmi le laurylméthicone copolyol, le cétyl diméthicone copolyol, un mélange de diméthicone copolyol et cyclométhicone ou un mélange de cétyl diméthicone copolyol avec du polyglycéryl-4 isostéarate et de l’hexyl laurate.

**3.** Composition selon l’une des revendications 1 ou 2 **caractérisée en ce qu’**elle contient également un co-émulsionnant ayant une HLB supérieure à 6.

**4.** Composition selon la revendication 3 **caractérisée en ce que** le co-émulsionnant est le cétéareth-20.

5. Composition selon l'une des revendications 1 à 4 **caractérisée en ce que** la proportion volumique de glycol, par rapport au volume total de la phase dispersée, se situe entre 10 et 100%.

6. Composition selon l'une des revendications 1 à 5 **caractérisée en ce que en ce que** la phase dispersée comprend au moins un glycol choisi parmi le propylène glycol, l'hexylène glycol et le dipropylène glycol.

7. Composition selon l'une des revendications 1 à 6 **caractérisée en ce que** l'activité en eau de la phase hydrophile dispersée, qui correspond au rapport de la pression de vapeur d'eau de la phase hydrophile sur la pression de vapeur de l'eau pure à la même température, est inférieure à 0,85.

8. Composition selon l'une des revendications 1 à 7, **caractérisée en ce que** la fraction volumique de la phase hydrophile dispersée est comprise entre 10 et 90% par rapport au volume total de l'émulsion.

9. Composition qui est une émulsion triple de type phase hydrophile/phase lipophile/phase hydrophile comportant une phase hydrophile externe, et une phase lipophile constituant avec une phase hydrophile interne une émulsion Inverse, **caractérisée en ce que** l'émulsion inverse est une composition telle que définie dans l'une des revendications 1 à 8.

10. Composition selon l'une des revendications 1 à 9 **caractérisée en ce qu'**elle contient également un ou plusieurs actifs choisis parmi les isoflavonoïdes, les inhibiteurs de métalloprotéinase, les caroténoïdes, les composés anti-glycation, les inhibiteurs de NO-synthase, les vitamines, les agents desquamants, les composés augmentant la synthèse des glycosaminoglycanes, les composés anti-irritants, les composés diminuant l'irritation d'origine neurogène, les composés myorelaxants et les dépigmentants.

11. Composition selon l'une des revendications 1 à 10 **caractérisée en ce qu'**elle contient de la DHEA.

12. Composition selon la revendication 1 **caractérisée en ce que** le dérivé de la DHEA est la 3-O-acetyl-7-benzoloxy-déhydroéplandrostérone.

13. Composition selon l'une des revendications 1 à 12 **caractérisée en ce qu'**elle contient entre 0,001 et 20 % en poids de DHEA et/ou ses précurseurs ou dérivés chimiques et/ou biologiques, par rapport au poids total de la composition.

14. Composition selon la revendication 13 **caractérisée en ce qu'**elle contient entre 0,2 et 4 % en poids de DHEA et/ou ses précurseurs ou dérivés chimiques et/ou biologiques, par rapport au poids total de la composition.

15. Utilisation cosmétique d'une composition selon l'une quelconque des revendications 1 à 14 dans la prévention et/ou le traitement des signes du vieillissement cutané chronologique ou actinique.

16. Utilisation cosmétique d'une composition selon l'une des revendications 1 à 14 pour traiter et/ou protéger la peau, les muqueuses ou les fibres kératiniques.

17. Utilisation cosmétique d'une composition selon l'une des revendications 1 à 14 pour prévenir ou traiter la canitie.

18. Utilisation cosmétique d'une composition selon l'une des revendications 1 à 14 pour atténuer les taches pigmentaires.

19. Utilisation d'une composition selon l'une quelconque des revendications 1 à 14 pour fabriquer une préparation pharmaceutique destinée à prévenir ou à traiter l'atrophie de la peau ou des muqueuses.

20. Utilisation d'une composition selon l'une des revendications 1 à 14 pour fabriquer une préparation pharmaceutique destinée à prévenir ou à traiter l'atrophie vulvaire ou vaginale.

21. Composition selon l'une des revendications 1 à 14 à titre de médicament.

## Claims

1. Composition containing DHEA and/or its precursors chosen from Δ5-pregnenolone, 17α-hydroxypregnenolone,

diosgenin, hecogenin, hecogenin acetate, smilagenin and sarsapogenin, or its chemical and/or biological derivatives chosen from 7-α-OH-DHEA, 7-β-OH-DHEA, 7-keto-DHEA, Δ5-androstene-3,17-diol and Δ4-androstene-3,17-dione, and the DHEA derivatives of formula (1):

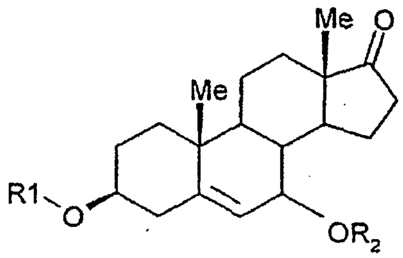

(1)

in which:

$R_1$ and $R_2$ are independently chosen from:

- a linear, branched or cyclic, saturated or unsaturated, $C_1$-$C_{12}$ alkyl group which may optionally contain one or more heteroatoms, and may be optionally substituted with one or more groups chosen from -OR' and/or -SR' and/or -COOR' and/or -NR'R' and/or halogen and/or sulfate and/or phosphate and/or aryl and/or heterocycle, it being possible for said heterocycle to be advantageously chosen from an indole, a pyrimidine, a piperidine, a morpholine, a pyran, a furan, a piperazine, a pyridine;
- an alkylcarbonyl group, in which the $C_1$-$C_{24}$ alkyl part is linear, branched or cyclic, saturated or unsaturated, and is optionally substituted with one or more groups chosen from -OR' and/or -SR' and/or -COOR' and/or -NR'R' and/or halogen and/or sulfate and/or phosphate and/or aryl and/or heterocycle, it being possible for said heterocycle to be advantageously chosen from an indole, a pyrimidine, a piperidine, a morpholine, a pyran, a furan, a piperazine, a pyridine;
- an arylcarbonyl, preferably a phenylcarbonyl, group or an arylalkylcarbonyl, preferably a benzylcarbonyl, group optionally substituted with one or more groups -OR' and/or -SR' and/or -COOR' and/or -NR'R' and/or halogen and/or aryl and/or heterocycle;
- a group O=P(OH)OR';
- a group $(O)_2SOR'$;
- a trialkylsilyl ($SiR'_3$) group in which the 3 groups R' may be identical or different;
- a carbonyloxyalkyl (R'OCO) group;
- a carbonylaminealkyl (R'NHCO) group;

in which R' is chosen from a hydrogen atom, a linear, branched or cyclic, saturated or unsaturated, $C_1$-$C_{12}$, preferably $C_1$-$C_6$, alkyl group which may optionally contain one or more heteroatoms, optionally functionalized with one or more groups -OR", -COOR", halogen, -NR"R"; or with an aryl, preferably a phenyl, group optionally functionalized with one or more groups -OR", -COOR", halogen, or -NR"R";
R" representing a hydrogen atom, a linear, branched or cyclic, saturated or unsaturated, preferably $C_1$-$C_6$, alkyl chain, it being understood that in each of the groups -NR'R' and -NR"R", the substituents R', respectively R", are identical or different,
**characterized in that** the composition is an invert emulsion containing a glycolic or hydroglycolic dispersed hydrophilic phase, a lipophilic continuous phase and a silicone emulsifier having an HLB of between 2 and 7, and **in that** the DHEA and/or its precursors or its chemical and/or biological derivatives is solubilized in the glycolic or hydroglycolic dispersed hydrophilic phase.

**2.** Composition according to Claim 1, **characterized in that** the emulsifier is chosen from lauryl methicone copolyol, cetyl dimethicone copolyol, a mixture of dimethicone copolyol and cyclomethicone or a mixture of cetyl dimethicone copolyol with polyglyceryl-4 isostearate and hexyl laurate.

**3.** Composition according to either of Claims 1 and 2, **characterized in that** it also contains a coemulsifier having an HLB of greater than 6.

4. Composition according to Claim 3, **characterized in that** the coemulsifier is ceteareth-20.

5. Composition according to one of Claims 1 to 4, **characterized in that** the volume proportion of glycol, relative to the total volume of the dispersed phase, is between 10 and 100%.

6. Composition according to one of Claims 1 to 5, **characterized in that** the dispersed phase comprises at least one glycol chosen from propylene glycol, hexylene glycol and dipropylene glycol.

7. Composition according to one of Claims 1 to 6, **characterized in that** the water activity of the dispersed hydrophilic phase, which corresponds to the ratio of the vapour pressure of water in the hydrophilic phase to the vapour pressure of pure water at the same temperature, is less than 0.85.

8. Composition according to one of Claims 1 to 7, **characterized in that** the volume fraction of the dispersed hydrophilic phase is between 10 and 90% relative to the total volume of the emulsion.

9. Composition which is a triple emulsion of the hydrophilic phase/lipophilic phase/hydrophilic phase type containing an external hydrophilic phase, and a lipophilic phase constituting, with an inner hydrophilic phase, an invert emulsion, **characterized in that** the invert emulsion is a composition as defined in one of Claims 1 to 8.

10. Composition according to one of Claims 1 to 9, **characterized in that** it also contains one or more active agents chosen from isoflavonoids, metalloproteinase inhibitors, carotenoids, antiglycation compounds, NO-synthase inhibitors, vitamins, desquamating agents, compounds increasing the synthesis of glycosaminoglycans, antiirritant compounds, compounds reducing irritation of neurogenic origin, muscle-relaxing compounds and depigmenting agents.

11. Composition according to one of Claims 1 to 10, **characterized in that** it contains DHEA.

12. Composition according to Claim 1, **characterized in that** the DHEA derivative is 3-O-acetyl-7-benzoyloxydehydroepiandrosterone.

13. Composition according to one of Claims 1 to 12, **characterized in that** it contains between 0.001 and 20% by weight of DHEA and/or its chemical and/or biological precursors or derivatives, relative to the total weight of the composition.

14. Composition according to Claim 13, **characterized in that** it contains between 0.2 and 4% by weight of DHEA and/or its chemical and/or biological precursors or derivatives, relative to the total weight of the composition.

15. Cosmetic use of a composition according to any one of Claims 1 to 14 in the prevention and/or treatment of the signs of chronological or actinic skin aging.

16. Cosmetic use of a composition according to one of Claims 1 to 14, for treating and/or protecting the skin, the mucous membranes or the keratinous fibers.

17. Cosmetic use of a composition according to one of Claims 1 to 14, for preventing or treating canities.

18. Cosmetic use of a composition according to one of Claims 1 to 14, for attenuating pigmented spots.

19. Use of a composition according to any one of Claims 1 to 14, for manufacturing a pharmaceutical preparation intended for preventing or treating atrophy of the skin or of the mucous membranes.

20. Use of a composition according to one of Claims 1 to 14, for manufacturing a pharmaceutical preparation intended for preventing or treating vulvar or vaginal atrophy.

21. Composition according to one of Claims 1 to 14, as a medicament.

**Patentansprüche**

1. Zusammensetzung, die DHEA und/oder seine Vorläufer, die unter $\Delta$5-Pregnenolon, 17$\alpha$-Hydroxypregnenolon, Dios-

genin, Hecogenin, Hecogeninacetat, Smilagenin und Sarsapogenin ausgewählt sind, oder chemische und/oder biologische Derivate enthält, die unter 7-α-OH-DHEA, 7-β-OH-DHEA, 7-Keto-DHEA, Δ5-Androsten-3,17-diol, Δ4-Androsten-3,17-dion und DHEA-Derivaten der folgenden Formel (1) ausgewählt sind:

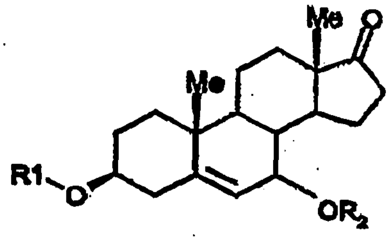

(1)

wobei in der Formel: sind unter:

$R_1$ und $R_2$ unabhängig voneinander ausgewählt sind unter:

- einer geradkettigen, verzweigten oder cyclischen, gesättigten oder ungesättigten $C_{1-12}$-Alkylgruppe, die gegebenenfalls ein oder mehrere Heteroatome enthalten und gegebenenfalls mit einer oder mehreren Gruppen substituiert sein kann, die ausgewählt sind unter -OR' und/oder -SR' und/oder -COOR' und/oder NR'R' und/oder Halogen und/oder Sulfat und/oder Phosphat und/oder Aryl und/oder einem Heterocyclus, wobei der Heterocyclus vorteilhaft unter Indolen, Pyrimidinen, Piperidinen, Morpholinen, Pyranen, Furanen, Piperazinen und Pyridinen ausgewählt sein kann;
- einer Alkylcarbonylgruppe, bei der der $C_{1-24}$-Alkylteil gesättigt oder ungesättigt, geradkettig, verzweigt oder cyclisch vorliegen und gegebenenfalls mit einer oder mehreren Gruppen substituiert sein kann, die unter -OR' und/ oder -SR' und/ oder -COOR' und/ oder -NR'R' und/ oder Halogen und/ oder Sulfat und/ oder Phosphat und/ oder Aryl und/ oder einem Heterocyclus ausgewählt sein kann, wobei der Heterocyclus vorteilhaft unter Indolen, Pyrimidinen, Piperidinen, Morpholinen, Pyranen, Furanen, Piperazinen und Pyridinen ausgewählt sein kann;
- einer Arylcarbonylgruppe und vorzugsweise einer Phenylcarbonylgruppe oder einer Arylalkylcarbonylgruppe, vorzugsweise einer Benzylcarbonylgruppe, die gegebenenfalls mit einer oder mehreren Gruppen -OR' und/ oder -SR' und/oder -COOR' und/oder NR'R' und/oder Halogen und/oder Aryl und/oder einem Heterocyclus substituiert sein können;
- einer Gruppe O=P(OH)OR';
- einer Gruppe $(O)_2SOR'$;
- einer Trialkylsilylgruppe ($SiR'_3$), worin die 3 Gruppen R' gleich oder verschieden sein können;
- einer Carbonyloxyalkylgruppe (R'OCO);
- einer Carbonylaminalkylgruppe (R'NHCO);

worin die Gruppen R' unter Wasserstoff, einer gesättigten oder ungesättigten, geradkettigen, verzweigten oder cyclischen Alkylgruppe mit 1 bis 12 Kohlenstoffatomen und vorzugsweise 1 bis 6 Kohlenstoffatomen, die gegebenenfalls ein oder mehrere Heteroatome enthalten kann und gegebenenfalls mit einer oder mehreren Gruppen -OR", -COOR", Halogen, -NR"R"; oder einer Arylgruppe und vorzugsweise einer Phenylgruppe funktionalisiert ist, die gegebenenfalls mit einer oder mehreren Gruppen -OR", -COOR", Halogen oder -NR"R" funktionalisiert ist;
wobei R" ein Wasserstoffatom oder eine gesättigte oder ungesättigte, lineare, verzweigte oder cyclische Alkylgruppe mit vorzugsweise 1 bis 6 Kohlenstoffatomen bedeutet,
mit der Maßgabe, dass in den Gruppen -NR'R' und -NR"R" die Substituenten R' bzw. R" jeweils gleich oder verschieden sind,
**dadurch gekennzeichnet, dass** die Zusammensetzung eine inverse Emulsion ist, die eine dispergierte hydrophile Glycolphase oder Hydroglycolphase, eine kontinuierliche lipophile Phase und einen siliconierten Emulgator mit einem HLB-Wert von 2 bis 7 enthält, und **dadurch**, dass das DHEA und/oder seine Vorläufer oder chemischen und/ oder biologischen Derivate in der dispergierten hydrophilen Glycolphase oder Hydroglycolphase solubilisiert sind.

2. Zusammensetzung nach Anspruch 1, **dadurch gekennzeichnet, dass** der Emulgator unter Laurylmethiconcopolyol, Cetyldimethiconcopolyol, einem Gemisch aus Dimethiconcopolyol und Cyclomethicon oder einem Gemisch

aus Cetyldimethiconcopolyol und Tetraglycerylisostearat und Hexyllaurat ausgewählt ist.

3. Zusammensetzung nach einem der Ansprüche 1 oder 2, **dadurch gekennzeichnet, dass** der Coemulgator einen HLB-Wert über 6 aufweist.

4. Zusammensetzung nach Anspruch 3, **dadurch gekennzeichnet, dass** der Coemulgator das Ceteareth-20 ist.

5. Zusammensetzung nach einem der Ansprüche 1 bis 4, **dadurch gekennzeichnet, dass** der Volumenanteil an Glycol, bezogen auf das Gesamtvolumen der dispergierten Phase, im Bereich von 10 bis 100 % liegt.

6. Zusammensetzung nach einem der Ansprüche 1 bis 5, **dadurch gekennzeichnet, dass** die dispergierte Phase mindestens ein Glycol enthält, das unter Propylenglycol, Hexylenglycol und Dipropylenglycol ausgewählt ist.

7. Zusammensetzung nach einem der Ansprüche 1 bis 6, **dadurch gekennzeichnet, dass** die Wasseraktivität der dispergierten hydrophilen Phase, die dem Verhältnis des Dampfdrucks des Wassers in der hydrophilen Phase und des Dampfdrucks von reinem Wasser bei gleicher Temperatur entspricht, unter 0,85 liegt.

8. Zusammensetzung nach einem der Ansprüche 1 bis 7, **dadurch gekennzeichnet, dass** der Volumenanteil der dispergierten hydrophilen Phase im Bereich von 10 bis 90 %, bezogen auf das Gesamtvolumen der Emulsion, liegt.

9. Zusammensetzung, bei der es sich um eine Dreifachemulsion vom Typ hydrophile Phase/lipophile Phase/hydrophile Phase handelt, die eine äußere hydrophile Phase und eine lipophile Phase aufweist, die mit einer inneren hydrophilen Phase eine inverse Emulsion darstellt, **dadurch gekennzeichnet, dass** die inverse Emulsion eine Zusammensetzung nach einem der Ansprüche 1 bis 8 ist.

10. Zusammensetzung nach einem der Ansprüche 1 bis 9, **dadurch gekennzeichnet, dass** sie auch einen oder mehrere Wirkstoffe enthält, die unter den Isoflavonoiden, Metalloproteinase-Inhibitoren, Carotinoiden, Anti-Cxlycationswirkstoffen, NO-Synthase-Inhibitoren, Vitaminen, abschuppenden Wirkstoffen, Verbindungen, die die Synthese von Glycosaminoglycanen fördern, reizlindernden Verbindungen, Verbindungen, die die Irritation neurogenen Ursprungs vermindern, muskelrelaxierenden Verbindungen und depigmentierenden Wirkstoffen ausgewählt sind.

11. Zusammensetzung nach einem der Ansprüche 1 bis 10, **dadurch gekennzeichnet, dass** sie DHEA enthält.

12. Zusammensetzung nach Anspruch 1, **dadurch gekennzeichnet, dass** es sich bei dem DHEA-Derivat um das 3-O-Acetyl-7-benzoyloxydehydroepiandrosteron handelt.

13. Zusammensetzung nach einem der Ansprüche 1 bis 12, **dadurch gekennzeichnet, dass** sie 0,001 bis 20 Gew.-% DHEA und/oder seiner Vorläufer oder chemischen und/oder biologischen Derivate, bezogen auf das Gesamtgewicht der Zusammensetzung, enthält.

14. Zusammensetzung nach Anspruch 13, **dadurch gekennzeichnet, dass** sie 0,2 bis 4 Gew.-% DHEA und/oder seiner Vorläufer oder chemischen und/oder biologischen Derivate, bezogen auf das Gesamtgewicht der Zusammensetzung, enthält.

15. Kosmetische Verwendung einer Zusammensetzung nach einem der Ansprüche 1 bis 14 für die Vorbeugung und/oder Behandlung von altersbedingten oder aktinischen Anzeichen der Hautalterung.

16. Kosmetische Verwendung einer Zusammensetzung nach einem der Ansprüche 1 bis 14 zur Behandlung und/oder zum Schutz der Haut, der Schleimhäute oder der Keratinfasern.

17. Kosmetische Verwendung einer Zusammensetzung nach einem der Ansprüche 1 bis 14 zur Vorbeugung oder Behandlung von Haarergrauen.

18. Kosmetische Verwendung einer Zusammensetzung nach einem der Ansprüche 1 bis 14, um Pigmentflecken abzumildern.

19. Verwendung einer Zusammensetzung nach einem der Ansprüche 1 bis 14 für die Herstellung eines pharmazeutischen Präparats, das dazu vorgesehen ist, die Atrophie der Haut oder der Schleimhäute zu behandeln oder ihr

vorzubeugen.

**20.** Verwendung einer Zusammensetzung nach einem der Ansprüche 1 bis 14 für die Herstellung eines pharmazeutischen Präparats, das dazu vorgesehen ist, die vulvare oder vaginale Atrophie zu behandeln oder ihr vorzubeugen.

**21.** Zusammensetzung nach einem der Ansprüche 1 bis 14 als Arzneimittel.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- JP 07196467 B
- US 4496556 A
- US 5843932 A
- FR 2803513
- EP 1092423 A
- WO 0054763 A
- FR 2777194
- US 5412004 A
- EP 0268164 A


### Littérature non-brevet citée dans la description


- **CHANG et al.** *J. Pharm. Sci.,* 1995, vol. 84, 1169-1179
- **MOTOYOSHI et al.** *Cosmet and toiletries,* 1984, vol. 99, 83-89
- **KABALNOV et al.** *J.ColloTd and interface Science,* 1987, vol. 118, 590-597